# EUROPEAN PATENT SPECIFICATION

(11) **EP 0 746 291 B1**
(45) Date of publication and mention of the grant of the patent: **11.04.2001**
(21) Application number: 94905956.2
(22) Date of filing: 23.12.1993
(51) Int. Cl.: A61F 13/00

(54) **DISPOSABLE WOUND DRESSING PERMITTING NON-INVASIVE EXAMINATION**
WEGWERFBARER WUNDVERBAND ZUR NICHTINVASIVEN PRÜFUNG
PANSEMENT JETABLE POUR UNE BLESSURE PERMETTANT UN EXAMEN NON INVASIF

(43) Date of publication of application: 11.12.1996
(73) Proprietor: Hathman, Johnnie L., Culver City, CA 90230 (US)
(72) Inventor: Hathman, Johnnie L., Culver City, CA 90230 (US)
(74) Representative: Schwabe - Sandmair - Marx
(86) International application number: US9312577
(87) International publication number: WO9517146

(56) References cited:
- US-A- 4 399 816
- US-A- 4 470 410
- US-A- 4 641 643
- US-A- 4 709 695
- US-A- 5 086 763

## Description

### Technical Field

This invention relates to disposable bandages and surgical dressings for wounds and particularly to those wound dressings that may be employed in connection with recurrent observation, examination and repeated medication of wounds.

In the employment of the novel wound dressing described in this specification, it is intended that the wound under treatment be completely circumscribed by an opening in the dressing. As will become apparent, means are provided whereby the wound may be inspected and treated with medication without removal of the primary body of the dressing itself.

### Background Art

The prior art includes United States Patent Number. 3,026,874, issued March 27, 1962 to R.C. Stevens for WOUND SHIELD. The device provides controlled drainage for a wound and is characterized as easily sterilizable thus leading to the inference of non-disposability.

Another prior art device is described in the United States Patent Number 4,470,410, granted to E.M. Elliot for PROTECTIVE RETAINING DEVICE AND METHOD, issued September 11, 1984. The device described appears to be comparatively expensive and complicated to manufacture. It appears to have as its primary purpose the protection of the site of an intravenous catheter.

Yet another prior art device has been illustrated in the United States Patent Number 3,888,247, granted to C.B. Stenvall for FIRST AID BANDAGE,, issued June 10, 1975. The innovative device described in that patent provides that a lightly adhered breathable surgical tape that is first placed over the wound shall be left in contact therewith until after healing has been accomplished.

The patent entitled PROTECTIVE DEVICE by inventors Kohn et al bearing United States patent number 4,709,695 was issued on December 1, 1987, and contemplates a contrivance expected to be prepared by cutting to size for each application.

US-A-5086763 discloses a disposable protective recloseable wound dressing having an epidermal adhesive tape strip base member for adhering a dressing to the skin of a user, the base member having a wound circumscribing aperture therein, a frame mounted about the aperture made from fuzz or felt material and a cover for the aperture having microhook material on an underside thereof for co-operating with the fuzz or felt material to close the aperture.

US-A-4399816 discloses a wound protector having a base member for adhesively attaching to the skin of a user, the base member having a wound circumscribing aperture therein, and a cover having an adhesive strip along one end thereof for securing the cover to the body and an opposite end of the cover having a portion applied with adhesive for releasably holding the cover in position over the aperture on the base member.

US-A-4641463 discloses a resealing skin bandage having a flexible body with an aperture therein for circumscribing a wound and a hinged cover member is formed from a folded-over member that has a lower portion adhered to the body and having an aperture in the lower portion thereof in alignment with the aperture in the body, and the upper portion of the hinged cover releasably closes the aperture. The fold of the cover acts as a hinge.

These prior art devices are commendable and show a truly creative spirit for their times and intended applications. The inventors and their inventions have contributed remarkably to the technology involved. However, these prior art structures do not include those combined elements of the invention described and submitted herewith that provide greater facility of use and ingenious arrangement of components that make the instant invention the high culmination in the art of disposable wound dressings that permit repeated, non-wound disturbing observations and recurring medications of a wound undergoing the healing process.

### DISCLOSURE OF THE INVENTION

According to a first aspect of this invention there is provided a disposable wound dressing as claimed in claim 1 herein.

According to a second aspect of this invention there is provided a disposable wound dressing as claimed in claim 2 herein.

In the instant invention, there is provided a disposable, inexpensive and easy to use wound dressing that expedites repetitious examination, observation and medication of a wound such as an injury resulting, in general, from a bruise, cut, laceration or the like. The inventive wound dressing contemplated by the device described envisions in succession, the initial sealing of a bandage covering and protecting the wound involved, the uninvasive unsealing of the bandage in such manner as to allow inspection of the wound under healing, observation of the wound, medication thereof as may be required, and the resealing of said bandage to again cover and protect the wound without loss of integrity of the sealing properties of the dressing.

### BRIEF DESCRIPTION OF DRAWINGS

Further advantages and features of the instant invention will be more fully apparent to those skilled in the art to which the invention pertains from the ensuing detailed description thereof, regarded in conjunction with the accompanying drawings wherein like reference numerals refer to like parts throughout and in which:
Figure 1 is an exploded, perspective view of a Disposable Wound Dressing Permitting Non-Invasive Examination useful in understanding the present invention.
Figure 2 is a perspective view of Figure 1 showing the dressing closed and latched.
Figure 3 is another perspective view of Figure 1 showing a medication pad in place and the dressing opened for inspection or the like.
Figure 4 is an exploded perspective view of another Disposable Wound Dressing Permitting Non-Invasive Examination and having a standoff member, the service of which will become apparent in the following discourse.
Figure 5 is a detailed perspective view showing the closure and latching of Figure 4 so as to effectuate the seal thereof.
Figure 6 is an open, perspective view of Figure 4 showing how it might be unsealed for inspection and possible replacement of a medication pad.
Figure 7 is an enlarged, detail cross sectional view of Figure 4 taken along the sight lines 7-7 of Figure 5.
Figure 8 is yet another Disposable Wound Dressing Permitting Non-Invasive Examination showing a unique stepped standoff member whose utility will be made apparent in the ensuing disclosure.
Figure 9 is an enlarged, detail cross sectional view of Figure 8 taken along the sight lines 9-9 of Figure 8 with the circumscribing cover member closed and latched so as to effect a seal.
Figure 10 is a perspective view of a first embodiment of the Disposable Wound Dressing Permitting Non-Invasive Examination having a tongue and groove latching mechanism in accordance with the second aspect of this invention.
Figure 11 is an enlarged detail cross sectional view taken along the sight lines 11-11 of Figure 10 with the circumscribing cover member closed so as to effect a seal.
Figure 12 is an exploded view of a further Disposable Wound Dressing Permitting Non-Invasive Examination showing a contourable peidermal adhesive tape member and additional advantages that will become apparent in the ensuing discourse.
Figure 13 is a perspective open view of a second embodiment of the Disposable Wound Dressing Permitting Non-Invasive Examination in accordance with the first aspect of this invention having unique characteristics to be explored in the following exposition.
Figure 14 is a perspective view of the embodiment of Figure 13 shown in a closed and sealed position.

### Best Mode for Carrying Out the Invention

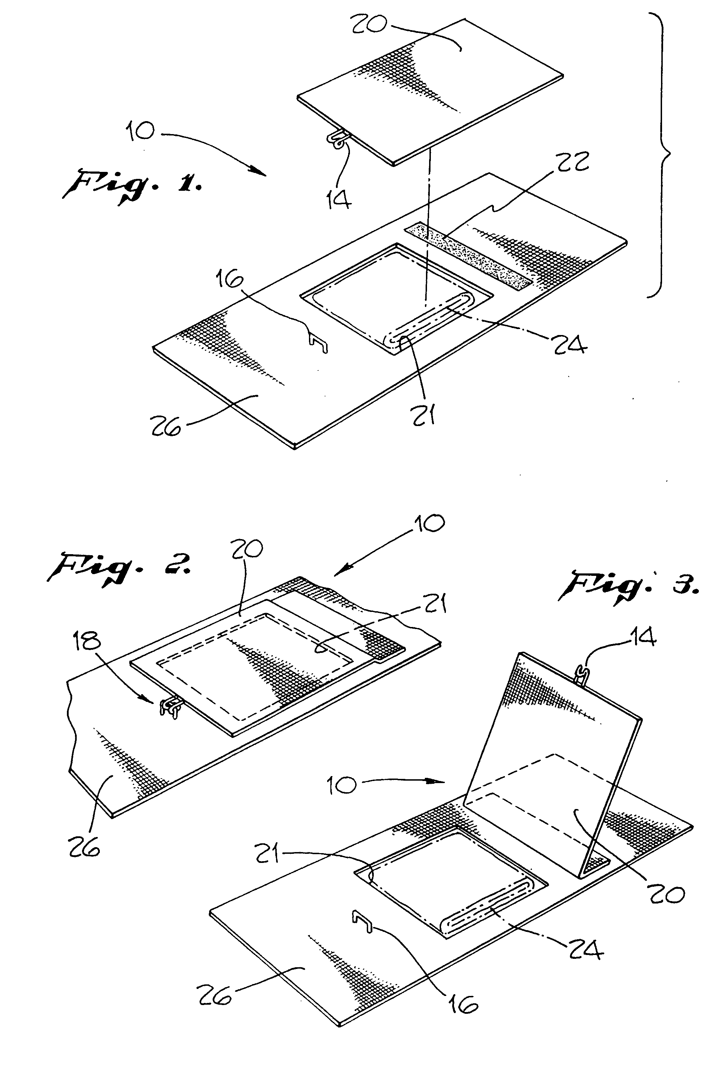

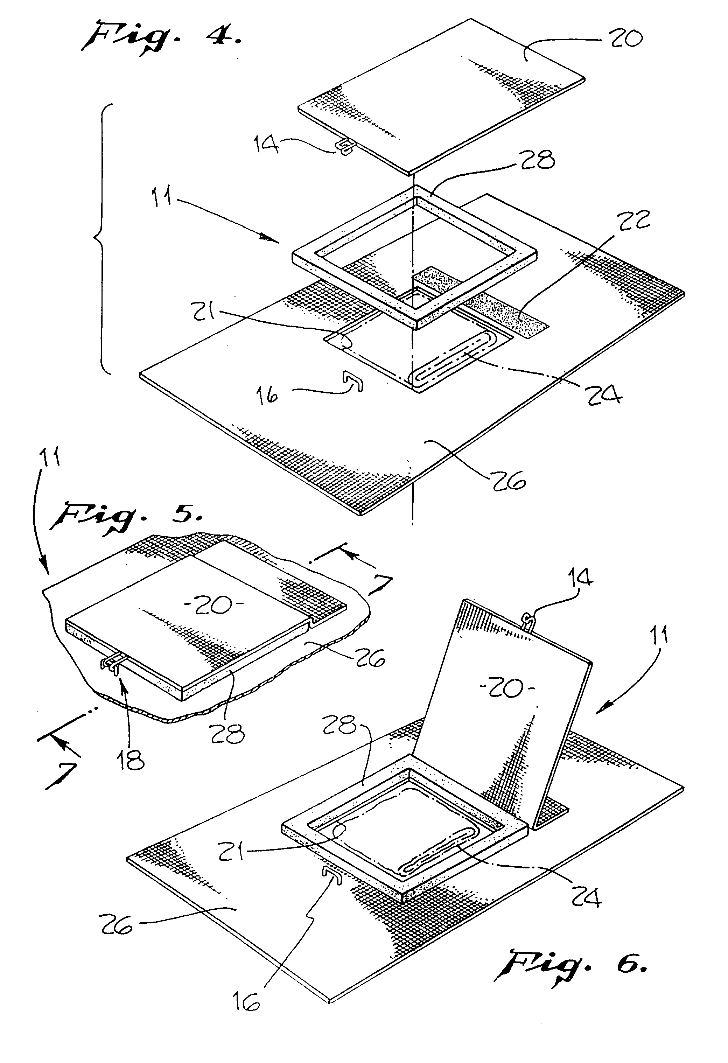

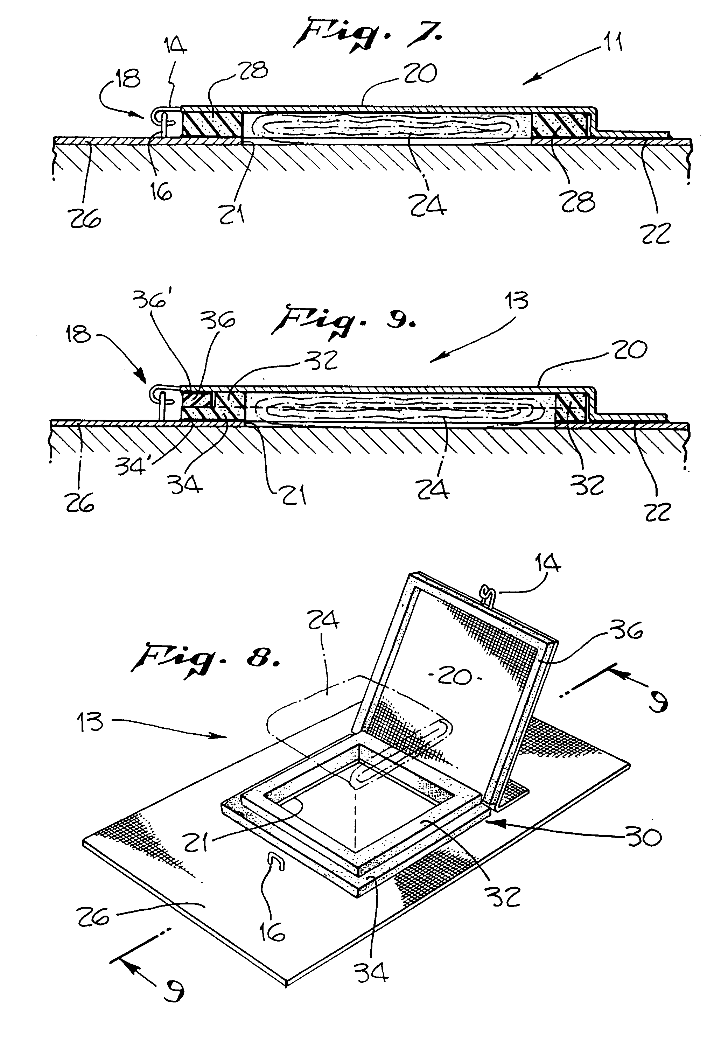

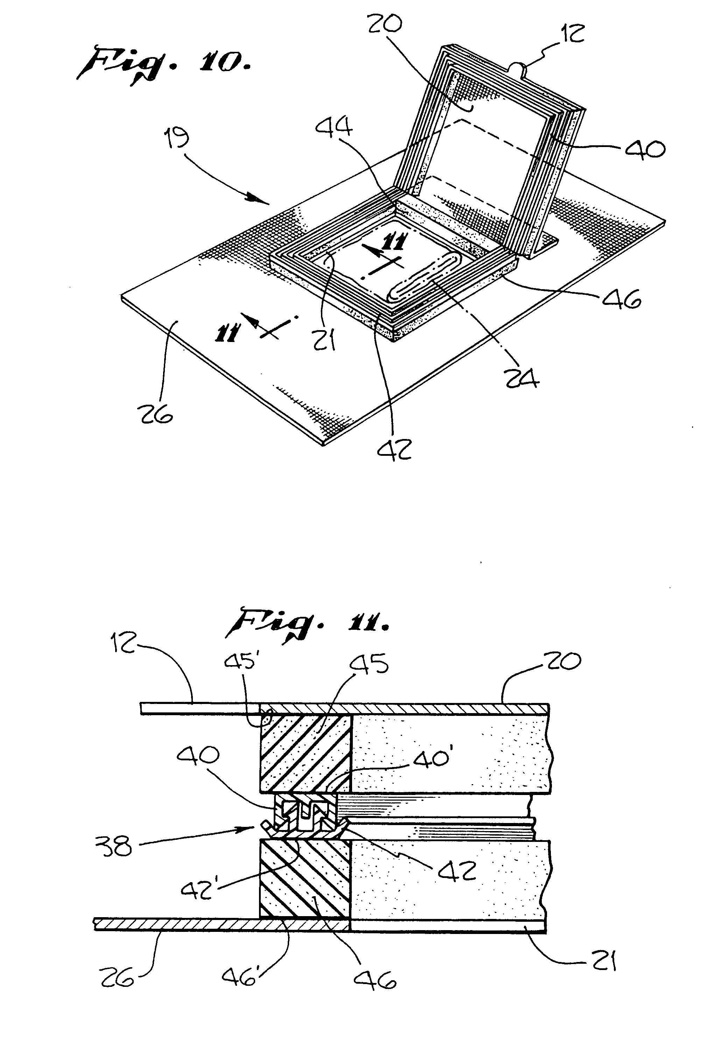

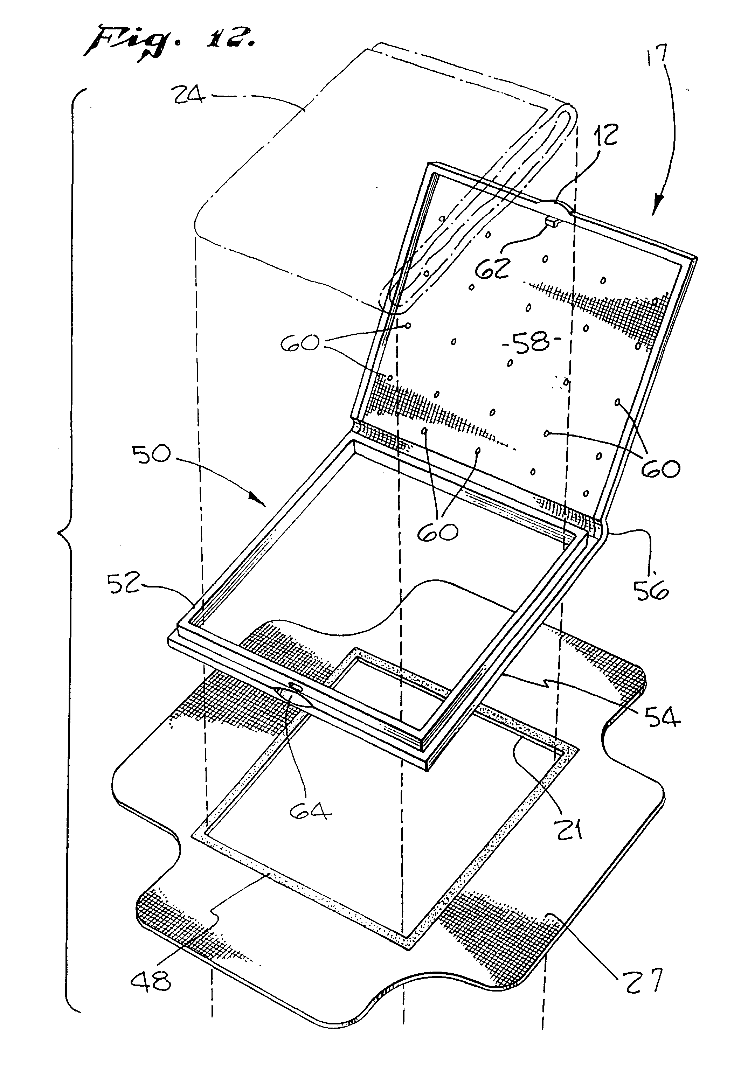

Referring to the drawings and to Figures 1 through 3 with greater particularity, the Disposable Wound Dressing Permitting Non-Invasive Examination is denoted generally by the numeral 10. Figure 1 shows the sealing cover member 20 prior to its secure attachment to adhesive strip 22 so as to form a hinge at one end of said sealing cover operative when opening or closing the dressing. An epidermal adhesive tape strip base member 26 adheres the wound dressing to the body skin of the patient and is so placed that the wound is circumscribed by the wound circumscribing aperture 21. An optional, replaceable pad which may be medicated or not as desired, is denoted by the numeral 24, and may be placed over the wound under treatment and within circumscribing aperture 21, however, medication may be applied or not by any other means desired and the illustration showing the pad 24 is not to be considered a limitation on the generality of the device. A hasp mechanism 18, Figure 2, having a latch and eye denoted respectively by numerals 14 and 16, effectuates positive closing and sealing of the dressing.

Figure 2 shows the sealing cover member 20 closed subsequent to its secure attachment at adhesive strip 22 and with latch and eye mechanism, respectively 14 and 16, engaged to form hasp 18 thus securing the sealing cover member 20 over wound circumscribing aperture 21 so as to protect the wound and secure the position of any medication applied.

Figure 3 displays the assembled device opened after application and illustrates the hinge action constraining sealing cover member 20 by means of the adhesive strip 22. The wound dressing may be opened, as illustrated, so as to inspect the wound under healing process, to apply medication to the wound or for any other purpose desired and then, after the accomplishment of the purpose for which the dressing was opened, resealed without loss of integrity of the original seal.

Directing attention now to Figures 4 through 7, another version, denoted generally by the numeral 11, of the Disposable Wound Dressing Permitting Non-Invasive Examination may be examined. Figure 4 is an exploded perspective view showing the constituent parts thereof prior to assembly. A sealing cover 20 is shown as to be attached by means of adhesive strip 22 to the epidermal adhesive tape base member 26. The cover 20 has a latch 14 intended for engagement with eye 16 on epidermal adhesive tape member 26. A medication pad 24 is shown in place upon a wound being treated; the wound has not been shown. Interposed between sealing cover 20 and epidermal adhesive tape base member 26 and dimensioned and positioned so as to be in registry with circumscribing aperture 21 in tape member 26, there is a standoff member 28. One of the purposes served by standoff member 28 is the alleviation of pressure upon a wound where such is necessary or desired in the treatment thereof.

Figure 5 illustrates the configuration of the dressing when closed by means of sealing cover 20 over standoff member 28 and secured by hasp 18. The integrity of the seal is intended to be maintained by means of sealing cover 20 as secured hingedly and adhesively by means of adhesive strip 26 and standoff member 28 acting cooperatively with hasp mechanism 18.

Figure 6 depicts the dressing opened subsequent to application to permit observation, medication or for any other purpose desired. Sealing cover 20 is shown in its hinge-open position while standoff member 28 is shown in registry with circumscribing aperture 21 in epidermal adhesive tape strip base member 26.

Figure 7 represents an enlarged cross sectional view taken along the sight lines 7 - 7 of Figure 5. The configuration of the sealing cover 20 with respect to the standoff member 28 may be more clearly understood in this enlarged view as may be the registration of standoff member 28 with circumscribing aperture 21. An additional point of view is also provided for explicating the cooperation of latch 14 with eye 16 to form hasp 18.

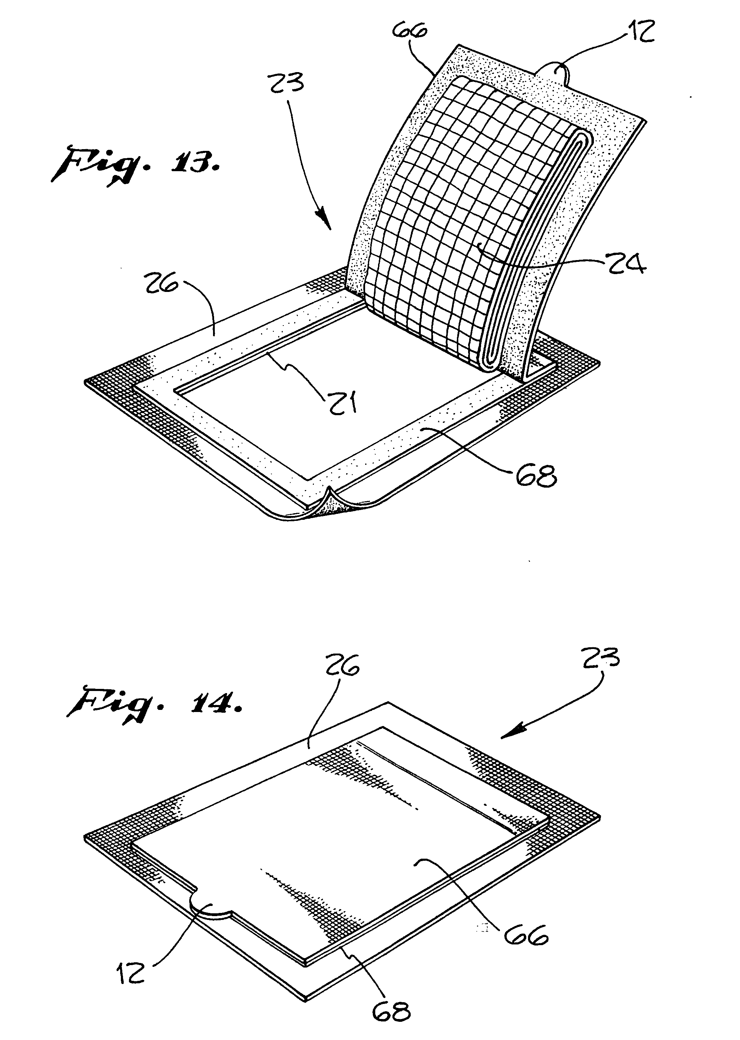

Yet another version of the Disposable Wound Dressing Permitting Non-Invasive Examination is represented in Figures 8 and 9 and denoted generally by the numeral 13. Figure 8 shows the dressing as applied after assembly and opened for inspection or for other purposes. The sealing cover 20 has a top circumscribing member 36 that acts in cooperation with a stepped standoff member 30 in effectuating a positive seal and a pressure reducing protection of a wound under treatment. Close examination reveals that top circumscribing member 36 is of the proper dimension and configuration so as to fit about a first stepped member 32 and onto and in abutment registration with a second stepped member 34.

Figure 9 represents an enlarged cross sectional view of this other version taken along the sight lines 9 - 9 of Figure 8. Detail there shows the adhesive attachment of stepped standoff member 30 to epidermal adhesive tape member 26 at second stepped member 34 by means of adhesive 34' and the adhesive attachment of top circumscribing member 36 to sealing cover 20 by means of adhesive 36'. Figure 9, of course, shows the dressing sealed and latched by means of hasp 18.

The invention is particularly depicted in Figures 10 and 11 in which the Disposable Wound Dressing Permitting Non-Invasive Examination is denoted generally by the numeral 19. A tongue-and-groove latching mechanism 38 is comprised of an upper latching member 40 which cooperates with a lower latching member 42 to bring about a seal of exceptionally high integrity. Sealing cover 20 supports the upper latching member 40 which is adhered to a top support member 45 by means of adhesive 40' which top support member 45 is in turn adhered to sealing cover 20 by adhesive 45'. In like manner, epidermal adhesive tape 26 supports the lower latching member 42 which is adhered to a bottom support member 46 by means of adhesive 42' which bottom support member 46 is in turn adhered to epidermal adhesive tape member 26 by means of adhesive 46'. A rear standoff member 44 is provided to enhance the structure and integrity of the seal effected by the cooperating members. A tab 12 is also provided in order to facilitate opening and closing the sealing cover 20 to provide access to the wound under treatment.

A further Disposable Wound Dressing Permitting Non-Invasive Examination is portrayed in exploded view in Figure 12 and denoted generally by the numeral 17. This further dressing features a contourable epidermal adhesive tape base member 27 that has an adhesive pattern 48 thereupon for the affixation of an adhered standoff member 50. Adhered standoff member 50 comprises a lower member 54 adapted to be adhered to adhesive pattern 48 and in registry with circumscribing aperture 21 in epidermal adhesive member 27 and an upper member 52 intended to be covered by attached covering member 58. Attached covering member 58 connects with standoff member 50 through resilient hinge 56. Covering member, 58 includes venting apertures 60 whose purpose is to provide circulation of ambient air to a wound under treatment. This further dressing provides a tab 12 and a latching mechanism comprising a latching member 62 adapted to be received into a latch recess 64 so as to effect positive closure of the covering member 58. Contourable epidermal adhesive base tape member 27 permits adherence of the dressing to irregularly shaped parts of the body and the forming around curved portions thereof of the dressing.

Figures 13 and 14 depict a second embodiment of the Disposable Wound Dressing Permitting Non-Invasive Examination denoted in general by the numeral 23. The circumscribing aperture 21, intended to circumscribe the wound under treatment, is situated in the epidermal adhesive tape strip base member 26. An adhesive backed sealing cover member 66 carries a pad 24 which may be medicated if desired, adhered to its wound facing surface. An adhesive cover matching strip 68 carried on the epidermal adhesive tape member 26 accomplishes positive sealing of the bandage when closed. A tab 12 is provided to facilitate opening and closing the cover 66.

### Industrial Applicability

The present invention finds application wherever surface wounds resulting from cuts, abrasions, bruises and stabs or the like occur. Most of the embodiments may be used in hospitals, homes, at athletic events, in the military and in all situations where injuries take place.

## Claims

1. A disposable wound dressing (23) permitting non-invasive examination of and access to a wound under treatment, comprising:
an epidermal adhesive tape strip base member (26) for adhering the dressing to the skin of a user of the dressing, the epidermal adhesive tape strip base member (26) having a wound circumscribing aperture (21);
a sealing cover member (66), the sealing cover member (66) comprising a first planar side and a second planar side, the second planar side having an adhesive disposed thereon;
a replaceable pad (24) placed over the wound under treatment and within the wound circumscribing aperture (21);
characterised by means (68) for securing the sealing cover member at one end to the epidermal adhesive tape strip base member (26), the means (68) for securing the sealing cover member at one end to the epidermal adhesive tape strip base member comprising a first portion of an adhesive cover matching strip, which is disposed about the wound circumscribing aperture, the first portion of the adhesive cover matching strip corresponding in dimension with the one end of the sealing cover member (66) in order to register with the one end of the sealing cover member (66), to thereby adhesively secure the sealing cover member (66) to the first portion of the adhesive cover matching strip;
means (68) for sealably closing the sealing cover member (66) to the epidermal adhesive tape strip base member (26) so as to cover the wound circumscribing aperture, the means for sealably closing the sealing cover member to the epidermal adhesive tape strip base member (26) so as to cover the wound circumscribing aperture comprising a second portion of the adhesive cover matching strip, the second portion of the adhesive cover matching strip corresponding in dimensions with both the wound circumscribing aperture and with a portion of the second planar side of the sealing cover member in order to register with the second planar side of the sealing cover member, to thereby sealably close the sealing cover member (66) to the epidermal adhesive tape strip base member (26) so as to cover the wound circumscribing aperture (21).

2. A disposable wound dressing (19) permitting non-invasive examination of and access to a wound under treatment, comprising:
a) an epidermal adhesive tape strip base member (26) for adhering the dressing to the skin of a user of the dressing, the epidermal adhesive tape strip base member (26) having a wound circumscribing aperture (21);
b) a sealing cover member (20);
c) means for securing the sealing cover member (20) at one end to the epidermal adhesive tape strip base member (26); and
d) characterised by means for sealably closing the sealing cover member (20) to the epidermal adhesive tape strip base member (26) so as to cover the wound circumscribing aperture (21), the means for sealably closing the sealing cover member (20) to the epidermal adhesive tape strip base member (26) comprising a tongue and groove latching mechanism (38) surrounding a majority of the wound circumscribing aperture (21), the tongue and groove latching mechanism comprising:
i) an upper latching member (40) secured to the sealing cover member (20), the upper latching member (40) surrounding a majority of the wound circumscribing aperture (21); and
ii) a lower latching member (42) secured to the epidermal adhesive tape strip base member (26), the lower latching member (42) surrounding a majority of the wound circumscribing aperture (21).

3. The disposable wound dressing of Claim 2, wherein the disposable wound dressing further comprises:
a replaceable pad (24) placed over the wound under treatment and within the wound circumscribing aperture (21); and
a rear standoff member (44) disposed at one end of the circumscribing aperture (21) and adhesively secured to the epidermal adhesive tape strip base member (26).

4. The disposable wound dressing of claims 2 or 3, in which the means for sealably closing the sealing cover member (20) to the epidermal adhesive tape strip base member (26), comprises means (12) for grasping and sealably closing the upper latching member (40) secured to the sealing cover member (28) to the lower latching member (42) secured to the epidermal adhesive tape strip base member (26) so as to cover the wound circumscribing aperture (21).

5. The disposable wound dressing of any of claims 2 to 4, in which the means for securing the sealing cover member (20) at one end to the epidermal adhesive tape strip base member (26) is an adhesive strip.

6. The disposable wound dressing of any of claims 2 to 5, wherein the upper latching member (40) is secured to the sealing cover member (20) by means of a top support member (45), the top support member (45) being adhesively secured both to the sealing cover member (20) and to the upper latching member (40) of the tongue and groove latching mechanism (38); and
wherein the lower latching member (42) is secured to the epidermal adhesive tape strip base member (26) by means of a bottom support member (46), the bottom support member (46) being adhesively secured both to the epidermal adhesive tape strip base member (26) and to the lower latching member (42) of the tongue and groove latching mechanism (38).

## Patentansprüche

1. Wegwerfbarer Wundverband (23), welcher die nicht-invasive Untersuchung bzw. Prüfung einer zu behandelnden Wunde und den Zugriff hierauf ermöglicht, mit:
einem epidermischen bzw. Oberhaut-Haft- bzw. -Klebe-Band-Streifen-Basis-Element (26) zum Anheften bzw. Ankleben des Verbandes an die Haut eines Benutzers des Verbandes, wobei das epidermische Haft-Band-Streifen-Basis-Element (26) eine die Wunde umgebende bzw. umlaufende Öffnung (21) aufweist;
einem abdichtenden bzw. verschließenden Abdeckelement (66), wobei das abdichtende Abdeckelement (66) eine erste planare bzw. ebene Seite und eine zweite ebene Seite aufweist, wobei die zweite ebene Seite ein darauf angeordnetes Haft- bzw. Klebemittel aufweist;
einem ersetzbaren bzw. austauschbaren Kissen bzw. Kompresse (24), welche über der zu behandelnden Wunde und innerhalb der die Wunde umgebenden Öffnung (21) angeordnet ist;
**gekennzeichnet** durch eine Vorrichtung (68) zum Verbinden bzw. Sichern des abdichtenden bzw. schließenden Abdeckelements bei einem Ende an dem epidermischen Haft-Band-Streifen-Basis-Element (26), wobei die Vorrichtung (68) zum Befestigen bzw. Verbinden des schließenden Abdeckelements bei einem Ende mit dem epidermischen Haft-Band-Streifen-Basis-Element einen ersten Teil eines Haft-Abdeck-Pass-Streifens aufweist, welcher um der die Wunde umgebenden Öffnung herum angeordnet ist, wobei der erste Teil des haftenden Abdeck-Pass-(matching)-Streifens bezüglich seiner Abmessung bzw. Dimension mit dem einen Ende des verschließenden Abdeckelements (66) übereinstimmt, um mit dem einen Ende des dichtenden Abdeckelements (66) räumlich übereinstimmend (registered) zu sein, um dadurch durch ein Anhaften bzw. Verkleben das abdichtende Abdeckelement (66) mit dem ersten Teil des haftenden bzw. klebenden Abdeck-Pass-(matching)-Streifens zu verbinden;
einer Vorrichtung (68), zum dichtenden bzw. abschließenden Verschließen des schließenden Abdeckelements (66) mit dem epidermischen Haft-Band-Streifen-Basis-Element (26), um so die die Wunde umgebende Öffnung abzudecken, wobei die Vorrichtung zum abdichtenden Schließen des abdichtenden Abdeckelements an bzw. mit dem epidermischen Haft-Band-Streifen-Basis-Element (26), um die die Wunde umgebende Öffnung abzudecken, einen zweiten Teil des Haft-Abdeck-Pass-Streifens aufweist, wobei der zweite Teil des Haft-Abdeck-Pass-Streifens hinsichtlich seiner Dimensionen bzw. Abmessungen mit beiden übereinstimmt bzw. entspricht, mit der die Wunde umgebenden Öffnung und mit einem Teil der zweiten planaren bzw. ebenen Seite des abdichtenden Abdeckelements, um mit der zweiten ebenen Seite des abdichtenden Abdeckelements räumlich bzw. passgenau übereinzustimmen (registered), um hierdurch das abdichtende Abdeckelement (66) mit dem epidermischen Haft-Band-Streifen-Basis-Element (26) abdichtend zu verschließen bzw. abzudecken, um so die die Wunde umgebende Öffnung (21) abzudecken.

2. Wegwerfbarer Wundverband (19), welcher eine nicht-invasive Untersuchung bzw. Prüfung und einen Zugriff auf eine zu behandelnde Wund ermöglicht, mit:
a) einem epidermischen Haft- bzw. Klebe-Band-Streifen-Basis-Element (26) zum Anheften bzw. Kleben des Verbandes an bzw. auf die Haut eines Benutzers des Verbandes, wobei das epidermische Haft-Band-Streifen-Basis-Element (26) eine Wunde umgebende Öffnung (21) aufweist;
b) einem dichtenden bzw. abschließenden Abdeckelement (20);
c) einer Vorrichtung zum Verbinden bzw. Sichern des abdichtenden Abdeckelements (20) bei einem Ende mit dem epidermischen Haft-Band-Streifen-Basis-Element (26); und
d) **gekennzeichnet** durch eine Vorrichtung zum abdichtenden Abdecken bzw. Verschließen des abdichtenden Abdeckelements (20) mit dem epidermischen Haft-Band-Streifen-Basis-Element (26), um die die Wunde umgebende Öffnung (21) abzudecken, wobei die Vorrichtung zum abdichtenden Abschließen des abdichtenden Abdeckelements (20) an bzw. mit dem epidermischen Haft-Band-Streifen-Basis-Element (26) einen Nut-und-Feder-(tongue and groove)-Verschluss-(latching)-Mechanismus (38) aufweist, welcher einen Großteil bzw. Hauptteil der die Wunde umgebenden Öffnung (21) umgibt, wobei der Nut-und-Feder-Verschließmechanismus aufweist:
i) ein oberes Schließelement (40), welches mit dem abdichtenden bzw. abschließenden Abdeckelement (20) verbunden bzw. daran befestigt ist, wobei das obere Schließelement (40) einen Groß- bzw. Hauptteil der die Wunde umgebenden Öffnung (21) umgibt; und
ii) einem unteren Schließelement (42), welches mit dem epidermischen Haft-Band-Streifen-Basis-Element (26) verbunden bzw. daran befestigt ist, wobei das untere Schließelement (42) einen Großteil der die Wunde umgebenden Öffnung (21) umgibt.

3. Wegwerfbarer Wundverband nach Anspruch 2, wobei der wegwerfbare Wundverband weiter aufweist:
ein ersetzbares bzw. austauschbares Kissen bzw. Kompresse (pad) (24), welche über der zu behandelnden Wunde und innerhalb der die Wunde umgebenden Öffnung (21) angeordnet ist; und
ein rückseitiges hervorstehendes bzw. Abstands-(standoff)-Element (44), welches bei einem Ende der umgebenden Öffnung (21) angeordnet ist und haftend bzw. klebend mit dem epidermischen Haft-Band-Streifen-Basis-Element (26) verbunden ist.

4. Wegwerfbarer Wundverband nach einem der Ansprüche 2 oder 3, wobei die Vorrichtung zum abdichtenden Abdecken bzw. Verschließen des abdichtenden Abdeckelements (20) an bzw. mit dem epidermischen Haft-Band-Streifen-Basis-Element (26) eine Vorrichtung (12) aufweist zum Erfassen bzw. Greifen und dichtenden Schließen des oberen Verschlusselements (40), welches an dem abdichtenden Verschlusselement (28) befestigt ist, mit dem unteren Verschlusselement (42), welches mit dem epidermischen Haft-Band-Streifen-Basis-Element (26) verbunden ist, um so die die Wunde umgebende Öffnung (21) abzudecken.

5. Wegwerfbarer Wundverband nach einem der Ansprüche 2 bis 4, wobei die Vorrichtung zum Befestigen des abdichtenden Deckelements (20) bei einem Ende an bzw. bei dem epidermischen Haft-Band-Streifen-Basis-Element (26) ein Haft-bzw. Klebestreifen ist.

6. Wegwerfbarer Wundverband nach einem der Ansprüche 2 bis 5, wobei das obere Verriegelungs- bzw. Schließelement (40) mit dem abdichtenden Abdeckelement (20) verbunden ist mittels eines oberen Stütz- bzw. Trageelements (45), wobei das obere Stütz- bzw. Trageelement (45) haftend bzw. klebend an beiden befestigt ist, an dem abdichtenden Abdeckelement (20) und an dem oberen Verschluss- bzw. Verriegelungselement (40) des Nut-und-Feder-Verriegelungs- bzw. -Verschlussmechanismus (38); und
wobei das untere Verriegelungs- bzw. Verschlusselement (42) an dem epidermischen Haft-Band-Streifen-Basis-Element (26) befestigt ist mittels eines unteren Stütz- bzw. Tragelements (46), wobei das untere Stütz- bzw. Tragelement (46) haftend bzw. klebend an beiden befestigt ist, an dem epidermischen Haft-Band-Streifen-Basis-Element (26) und an dem unteren Verriegelungs- bzw. Verschlusselement (42) des Nut-und-Feder-Verriegelungs- bzw. -Verschlussmechanismus (38).

## Revendications

1. Pansement pour blessure jetable (23) permettant l'examen non invasif et l'accès à une blessure qui est traitée, comprenant :
un élément de base à bande adhésive épidermique (26) pour faire adhérer le pansement à la peau d'un utilisateur du pansement, l'élément de base à bande adhésive épidermique (26) comportant une ouverture entourant la blessure (21) ;
un élément de recouvrement étanche (66), l'élément de recouvrement étanche comprenant un premier côté plan et un deuxième côté plan, le deuxième côté plan comportant un adhésif disposé sur celui-ci ;
un tampon remplaçable (24) disposé sur la blessure qui est traitée et à l'intérieur de l'ouverture entourant la blessure ;
caractérisé par des moyens (68) pour fixer l'élément de recouvrement étanche à une extrémité à l'élément de base à bande adhésive épidermique (26), les moyens (68) pour fixer l'élément de recouvrement étanche à une extrémité à l'élément de base à bande adhésive épidermique comprenant une première partie d'une bande d'adaptation de recouvrement adhésive, qui est - disposée autour de l'ouverture entourant la blessure, la première partie de la bande d'adaptation de recouvrement adhésive ayant des dimensions correspondant à la première extrémité de l'élément de recouvrement étanche (66) de façon à être alignée avec la première extrémité de l'élément de recouvrement étanche (66), de façon à fixer ainsi par adhérence l'élément de recouvrement étanche (66) à la première partie de la bande d'adaptation de recouvrement adhésive ;
des moyens (68) pour fermer de façon étanche l'élément de recouvrement étanche (66) sur l'élément de base à bande adhésive épidermique (26) de façon à recouvrir l'ouverture entourant la blessure, les moyens pour fermer de façon étanche l'élément de recouvrement étanche sur l'élément de base à bande adhésive épidermique (26) de façon à recouvrir l'ouverture entourant la blessure comprenant une deuxième partie de la bande d'adaptation de recouvrement adhésive, la deuxième partie de la bande d'adaptation de recouvrement adhésive ayant des dimensions qui correspondent tout à la fois à l'ouverture entourant la blessure et à une partie du deuxième côté plan de l'élément de recouvrement étanche afin d'être alignée avec le deuxième côté plan de l'élément de recouvrement étanche, de façon à fermer ainsi de façon étanche l'élément de recouvrement étanche (66) sur l'élément de base à bande adhésive épidermique (26) de façon à recouvrir l'ouverture entourant la blessure (21).

2. Pansement pour blessure jetable (19) permettant l'examen non invasif et l'accès à une blessure qui est traitée, comprenant :
(a) un élément de base à bande adhésive épidermique (26) pour faire adhérer le pansement à la peau d'un utilisateur du pansement, l'élément de base à bande adhésive épidermique (26) comportant une ouverture entourant la blessure (21) ;
(b) un élément de recouvrement étanche (20) ;
(c) des moyens pour fixer l'élément de recouvrement étanche (20) à une extrémité à l'élément de base à bande adhésive épidermique (26) ; et
(d) caractérisé par des moyens pour fermer de façon étanche l'élément de recouvrement étanche (20) sur l'élément de base à bande adhésive épidermique (26), de façon à recouvrir l'ouverture entourant la blessure (21), les moyens pour fermer de façon étanche l'élément de recouvrement étanche (20) sur l'élément de base à bande adhésive épidermique (26) comprenant un mécanisme de verrouillage à languette et à rainure (38) entourant une majorité de l'ouverture entourant la blessure (21), le mécanisme de verrouillage à languette et à rainure comprenant :
i) un élément de verrouillage supérieur (40) fixé à l'élément de recouvrement étanche (20), l'élément de verrouillage supérieur (40) entourant une majorité de l'ouverture entourant la blessure (21) ; et
ii) un élément de verrouillage inférieur (42) fixé à l'élément de base à bande adhésive épidermique (26), l'élément de verrouillage inférieur (42) entourant une majorité de l'ouverture entourant la blessure (21).

3. Pansement pour blessure jetable selon la revendication 2, dans lequel le pansement pour blessure jetable comprend de plus :
un tampon remplaçable (24) disposé sur la blessure qui est traitée et à l'intérieur de l'ouverture entourant la blessure (21) ; et
un élément d'arrêt arrière (44) disposé à une extrémité de l'ouverture d'entourage (21) et fixé par adhérence à l'élément de base à bande adhésive épidermique (26).

4. Pansement pour blessure jetable selon les revendications 2 ou 3, dans lequel'les moyens pour fermer de façon étanche l'élément de recouvrement étanche (20) sur l'élément de base à bande adhésive épidermique (26) comprennent des moyens (12) pour saisir et fermer de façon étanche l'élément de verrouillage supérieur (40) fixé à l'élément de recouvrement étanche (28) sur l'élément de verrouillage inférieur (42) fixé à l'élément de base à bande adhésive épidermique (26) de façon à recouvrir l'ouverture entourant la blessure (21).

5. Pansement pour blessure jetable selon l'une quelconque des revendications 2 à 4, dans lequel les moyens pour fixer l'élément de recouvrement étanche (20) à une extrémité de l'élément de base à bande adhésive épidermique (26) sont une bande adhésive.

6. Pansement pour blessure jetable selon l'une quelconque des revendications 2 à 5, dans lequel l'élément de verrouillage supérieur (40) est fixé à l'élément de recouvrement étanche (20) au moyen d'un élément de support supérieur (45), l'élément de support supérieur (45) étant fixé par adhérence tout à la fois à l'élément de recouvrement étanche (20) et à l'élément de verrouillage supérieur (40) du mécanisme de verrouillage à languette et à rainure (38) ; et
dans lequel l'élément de verrouillage inférieur (42) est fixé à l'élément de base à bande adhésive épidermique (26) au moyen d'un élément de support inférieur (46), l'élément de support inférieur (46) étant fixé par adhérence tout à la fois à l'élément de base à bande adhésive épidermique (26) et à l'élément de verrouillage inférieur (42) du mécanisme de verrouillage à languette et à rainure (38).
